# EUROPÄISCHE PATENTANMELDUNG

(11) **EP 1 570 852 A2**
(43) Veröffentlichungstag der Anmeldung: **07.09.2005**
(21) Anmeldenummer: 04106125.0
(22) Anmeldetag: 10.09.2001
(51) Int. Cl.: A61K 33/00, A61P 9/02, A61P 25/04, A61P 25/20

(54) **Xenon als Arzneimittel**

(30) Priorität: 14.09.2000 DE 10045845
(62) Teilanmeldung aus: 01972033.3
(71) Anmelder: Air Liquide Deutschland GmbH, 40235 Düsseldorf (DE)
(72) Erfinder: Baumert, Jan-Hinrich, D 52062 Aachen (DE); Coppin, Arnold., B-9111Belsele (BE); Hecker, Klaus.E, D-50126 Bergheim (DE)
(74) Vertreter: Jäger, Gerhard Fred

(57) **Zusammenfassung**

Xenon oder Xenon-haltige Gase werden als Arzneimittel eingesetzt, insbesondere als Herz-Kreislaufmittel, blutdruckerhöhendes Mittel, Sedativum oder Analgetikum. Die Arzneimittel enthalten vorzugsweise subanästhetische Mengen von Xenon.

## Beschreibung

Die Erfindung betrifft ein Arzneimittel, insbesondere ein Herz-Kreislaufmittel, ein Blutdruck erhöhendes Mittel, ein Analgetikum und ein Sedativum.

Xenon wird als Inhalationsanästhesiemittel verwendet. Die narkotischen Wirkungen von Xenon sind seit den 40er Jahren bekannt. Eine Mischung von etwa 80% Xenon und 20% Sauerstoff wird als nahezu ideales Narkosegas betrachtet, das gegenüber den heute überwiegend verwendeten Narkosegasen zahlreiche Vorteile hat. Eine Übersicht über die anästhetischen und pharmakologischen Eigenschaften des Xenon findet sich in F. Giunta et al., "Xenon: a review of its anaesthetic and pharmacological properties", Applied Cardiopulmonary Pathophysiology 00: 1-9, 1996.

Kreislaufmittel sind im allgemeinen Pharmaka, die Kreislaufgefäße erweitern oder verengen. Catecholamine wie Dopamin werden zur Steigerung des arteriellen Blutdrucks eingesetzt.

Herkömmliche Inhalationsanästhesiemittel mit Ausnahme von Xenon haben eine mehr oder weniger ausgeprägte Blutdruck senkende Wirkung. Bisher geht man bei Xenon davon aus, daß Xenon den Blutdruck nicht beeinflußt (vgl. Giunta et al., oben zitiert).

Bekannte Herz-Kreislaufmittel, Blutdruck beeinflussende Mittel, Analgetika und Sedativa werden im Körper des Patienten metabolisiert und zeigen mehr oder weniger ausgeprägt Nebenwirkungen.

Der Erfindung liegt die Aufgabe zugrunde, ein Arzneimittel, insbesondere ein Herz-Kreislaufmittel, ein Blutdruckmittel, ein Analgetikum und Sedativum, bereitzustellen, das weniger Nebenwirkungen aufweist.

Es wurde gefunden, daß gasförmiges Xenon bei einer inhalativen Verabreichung, insbesondere in subanästhetischen Mengen, als ein Herz-Kreislaufmittel, als Analgetikum und/oder als Sedativum wirkt.

Gegenstand der Erfindung ist somit ein Arzneimittel mit den in Anspruch 1 beschriebenen Merkmalen.

Das Arzneimittel ist insbesondere ein Kreislaufmittel, ein Blutdruck erhöhendes Mittel, ein Analgetikum und ein Sedativum.

Das Arzneimittel besteht aus Xenon oder einem Xenon-haltigen Gasgemisch oder enthält Xenon oder ein Xenon-haltiges Gasgemisch. Das Arzneimittel ist vorzugsweise gasförmig und wird vorzugsweise durch Inhalation über die Lunge verabreicht. Das Arzneimittel ist daher vorzugsweise ein Inhalationsarzneimittel. Das Arzneimittel wird bei dem Menschen oder bei Säugetieren eingesetzt.

Das Arzneimittel dient vorzugsweise als Herz-Kreislaufmittel, z. B. als Mittel zur Kreislaufstabilisierung, insbesondere als Mittel zur Blutdruckerhöhung, vorteilhaft für eine antihypotensive Therapie. Reines Xenon oder Xenon-haltige Gasgemische werden besonders vorteilhaft als ein Arzneimittel zur gleichzeitigen Blutdruckerhöhung, Sedation und Analgesie, also einem Kombinationsmittel für die genannten Indikationen, verwendet.

Das Arzneimittel wird in der Regel als reines gasförmiges Xenon bereitgestellt. Zum Einsatz kommt das Arzneimittel in der Regel als ein die Atmung unterhaltendes Gasgemisch, das Xenon und Sauerstoff enthält. Das Arzneimittel kann auch als Gasgemisch bereitgestellt werden. Solche Gasgemische werden beispielsweise in der Notfallmedizin eingesetzt, wo Gasmisch- oder Gasdosiergeräte für den mobilen Einsatz zu aufwendig sind.

Das bereitgestellte Arzneimittel oder das direkt bei der Anwendung, insbesondere in unmittelbarer Nähe zum Patienten, hergestellte Arzneimittel ist beispielsweise ein Gasgemisch, das 5 bis 70 Vol.-% Xenon enthält (z. B. Rest Sauerstoff). Vorteilhaft enthält das Arzneimittel, das dem Patienten verabreicht wird, Xenon in subanästhetischen Mengen. Als subanästhetische Mengen von Xenon sind solche Mengen oder Konzentrationen von Xenon zu verstehen, die für eine Anästhesie nicht ausreichen. Das sind im allgemeinen Mengen bis zu 70 Vol.-% Xenon, vorzugsweise im Bereich von 5 bis 65 Vol.-%, besonders bevorzugt im Bereich von 25 bis 65 Vol.-%, insbesondere im Bereich von 35 bis 60 Vol.-%. Reines Xenon als Arzneimittel wird dementsprechend in den genannten Konzentrationen im Atemgas des Patienten dosiert. Das heißt das dem Patienten zugeführte Atemgas enthält beispielsweise 35 bis 60 Vol.-% oder 35 bis 55 Vol.-% Xenon. In besonderen Fällen kann eine Dosierung von Xenon in dem Atemgas mit einer niedrigen Konzentration, beispielsweise 5 bis 25 Vol.-% Xenon in dem Atemgas, vorteilhaft sein.

In Notfallsituationen kann es sinnvoll sein, Xenon in hoher Konzentration oder als reines Gas kurzzeitig zu verabreichen (z.B. während eines Atemzuges).

Die Gasgemische können neben Xenon ein oder mehrere Gase oder bei Körpertemperatur und Normaldruck gasförmige Stoffe enthalten. Verwendbare Gasgemische sind beispielsweise Xenon-Sauerstoff-Gasgemische oder Gasgemische von Xenon und einem oder mehrerer Inertgase wie Stickstoff oder einem Edelgas (z. B. Helium, Neon, Argon, Krypton) oder Xenon-Sauerstoff-Inertgas-Gasgemische. Die Beimischung eines Gases zum Xenon kann sehr vorteilhaft sein, wenn wenig Xenon in den Körper gebracht werden soll. Beispiele von Gasen oder Gasgemischen, die als Arzneimittel (z. B. Kreislaufmittel, insbesondere als Mittel zur Blutdruckerhöhung) eingesetzt werden, folgen: 1.) 100 Vol.-% Xenon; 2.) 70 Vol.-% Xenon / 30 Vol.-% Sauerstoff; 3.) 65 Vol.-% Xenon / 30 Vol.-% Sauerstoff / 5 Vol.-% Stickstoff; 4.) 65 Vol.-% Xenon / 35 Vol.-% Sauerstoff; 5.) 60 Vol.-% Xenon / 30 Vol.-% Sauerstoff / 10 Vol.-% Stickstoff; 6.) 60 Vol.-% Xenon / 35 Vol.-% Sauerstoff / 5 Vol.-% Stickstoff; 7.) 60 Vol.-% Xenon / 40 Vol.-% Sauerstoff; 8.) 55 Vol.-% Xenon / 25 Vol.-% Sauerstoff / 20 Vol.-% Stickstoff; 9.) 55 Vol.-% Xenon / 30 Vol.-% Sauerstoff /15 Vol.-% Stickstoff; 10.) 55 Vol.-% Xenon / 35 Vol.-% Sauerstoff / 10 Vol.-% Stickstoff; 11.) 55 Vol.-% Xenon / 40 Vol.-% Sauerstoff / 5 Vol.-% Stickstoff; 12.) 55 Vol.-% Xenon / 45 Vol.-% Sauerstoff; 13.) 50 Vol.-% Xenon / 50 Vol.-% Sauerstoff; 14.) 50 Vol.-% Xenon / 45 Vol.-% Sauerstoff / 5 Vol.-% Stickstoff; 15.) 50 Vol.-% Xenon / 40 Vol.-% Sauerstoff / 10 Vol.-% Stickstoff; 16.) 50 Vol.-% Xenon / 30 Vol.-% Sauerstoff / 20 Vol.-% Stickstoff; 17.) 50 Vol.-% Xenon / 25 Vol.-% Sauerstoff / 25 Vol.-% Stickstoff; 18.) 45 Vol.-% Xenon / 55 Vol.-% Sauerstoff; 19.) 45 Vol.-% Xenon / 50 Vol.-% Sauerstoff / 5 Vol. -% Stickstoff; 20.) 45 Vol.-% Xenon / 45 Vol.-% Sauerstoff / 10 Vol.-% Stickstoff; 21.) 45 Vol.-% Xenon / 40 Vol.-% Sauerstoff / 15 Vol.-% Stickstoff; 22.) 45 Vol.-% Xenon / 35 Vol.-% Sauerstoff / 20 Vol.-% Stickstoff; 23.) 45 Vol. -% Xenon / 30 Vol.-% Sauerstoff / 25 Vol.-% Stickstoff; 24.) 45 Vol.-% Xenon / 30 Vol.-% Sauerstoff / 25 Vol.-% Stickstoff; 25.) 40 Vol.-% Xenon / 30 Vol.-% Sauerstoff / 30 Vol.-% Stickstoff; 26.) 40 Vol.-% Xenon / 50 Vol.-% Sauerstoff / 10 Vol.-% Stickstoff; 27.) 35 Vol.-% Xenon / 25 Vol.-% Sauerstoff / 40 Vol.-% Stickstoff; 28.) 35 Vol.-% Xenon / 65 Vol.-% Sauerstoff; 29.) 30 Vol.-% Xenon / 70 Vol.-% Sauerstoff; 30.) 30 Vol.-% Xenon / 50 Vol.-% Sauerstoff / 20 Vol.-% Stickstoff; 31.) 30 Vol.-% Xenon / 30 Vol.-% Sauerstoff / 40 Vol.-% Stickstoff; 32.) 20 Vol.-% Xenon / 80 Vol.-% Sauerstoff; 33.) 20 Vol.-% Xenon / 30 Vol.-% Sauerstoff / 50 Vol.-% Stickstoff; 34.) 15 Vol.-% Xenon / 30 Vol.-% Sauerstoff / 55 Vol.-% Stickstoff; 35.) 15 Vol.-% Xenon / 50 Vol.-% Sauerstoff / 35 Vol.-% Stickstoff; 36.) 10 Vol.-% Xenon / 90 Vol.-% Sauerstoff; 37.) 10 Vol.-% Xenon / 50 Vol.-% Sauerstoff / 40 Vol.-% Stickstoff; 38.) 10 Vol.-% Xenon / 30 Vol.-% Sauerstoff / 60 Vol.-% Stickstoff; 39.) 10 Vol.-% Xenon / 25 Vol.-% Sauerstoff / 65 Vol.-% Stickstoff; 40.) 5 Vol.-% Xenon / 25 Vol.-% Sauerstoff / 70 Vol.-% Stickstoff; 41.) 5 Vol.-% Xenon / 30 Vol.-% Sauerstoff / 65 Vol.-% Stickstoff; 42.) 5 Vol.-% Xenon / 50 Vol.-% Sauerstoff / 45 Vol.-% Stickstoff; 43.) 5 Vol.-% Xenon / 30 Vol.-% Sauerstoff / 65 Vol.-% Stickstoff; 44.) 5 Vol.-% Xenon / 95 Vol.-% Sauerstoff.

Xenon oder ein Xenon-haltiges Gasgemisch dient vorzugsweise zur Herstellung eines Arzneimittels zur Blutdruckerhöhung, zur Herstellung eines Medikamentes zur Behandlung von Herz-Kreislauf-Instabilitäten, zur Herstellung eines gasförmigen Medikamentes zur Behandlung von Krankheiten des Herzens und/oder des Kreislaufes, zur Herstellung eines gasförmigen Medikamentes zur Behandlung von Herz-Kreislauf-Instabilitäten von Risikopatienten oder zur Herstellung eines gasförmigen Medikamentes zur Behandlung von Herz-Kreislauf-Instabilitäten bei Schilddrüsenoperationen, Lebertransplantationen, in der Gynäkologie, in der Orthopädie, in der plastischen Chirurgie oder in der Onkologie. Ferner dienen Xenon oder Xenon-haltige Gasgemische als Medikament zur Behandlung von Schock oder schockartigen Zuständen.

Weiter wird das Arzneimittel zur Behandlung von hypotonen Kreislaufzuständen, verursacht durch Herzinsuffizienz, Hypotonie, Hypovolemie oder Anästhetikumbedingter Hypotonie (z. B. einer Blutdruckabsenkung bei Narkose mit Lachgas oder einem volatilen Anästhetikum), eingesetzt.

Das Arzneimittel (z. B. Herz-Kreislaufmittel, Analgetikum und/oder Sedativum) wird präoperativ, intraoperativ oder postoperativ eingesetzt.

Besonders vorteilhaft wird das Arzneimittel, insbesondere das Herz-Kreislaufmittel, Analgetikum und/oder Sedativum, in der Intensivmedizin eingesetzt, wo z. B. über einen längeren Zeitraum ein Kreislaufmittel, Analgetikum und/oder Sedativum verabreicht werden muß, beispielsweise bei der Langzeitbeatmung und bei Polytrauma. Hier hat das Arzneimittel den besonderen Vorteil, nach derzeitigem Kenntnisstand keine Nebenwirkungen zu haben. Es bilden sich im Körper bei Verwendung von Xenon oder Xenon-haltigen Gasen als Arzneimittel keine Metabolite im Körper und es findet im Körper keine Anreicherung des Arzneimittels statt. Übliche Arzneimittel wie übliche Kreislaufmittel, Analgetika oder Sedativa können wegen auftretenden Nebenwirkungen maximal nur wenige Tage verabreicht werden und müssen dann abgesetzt werden. Die kombinierte analgetische, sedierende und kreislaufstabilisierende Wirkung des Arzneimittels ist in dieser Form bei anderen Arzneimitteln nicht bekannt.

Xenon als Inhalationsarzneimittel insbesondere zur Stabilisierung des Patienten (Mittel für Kreislauf, insbesondere für Kreislauf und Analgesie oder für Kreislauf, Analgesie und Sedierung) bei der Beatmung, insbesondere der Langzeitbeatmung, wird in der Regel als Gemisch von Xenon, Sauerstoff und gegebenenfalls einem oder mehreren weiteren Gasen (z.B. Stickstoff) eingesetzt. Xenon wird dabei in der Regel in subanästhetischen Konzentrationen im atembaren Gas (Atemgas) verabreicht. Insbesondere bei der Langzeitbeatmung ist die Verabreichung von atembaren Gasen mit einem Gehalt von 5 bis 45 Vol.-% Xenon, vorzugsweise 5 bis 40 Vol.-% Xenon, vorteilhaft. Bei der Langzeitbeatmung hat das atembare Gas beispielsweise einen Gehalt von 20 bis 30 Vol.-% Sauerstoff, wobei Sauerstoffgehalt bei Bedarf zeitweise z. B. 30 bis 95 Vol.-% Sauerstoff erhöht werden kann. Das restliche Gas in dem atembaren Gas besteht in der Regel aus Stickstoff oder einem anderen Inertgas. Xenonhaltige Gasgemische für die Beatmung bestehen beispielsweise aus 5 Vol.-% Xenon/30 Vol.-% Sauerstoff/Rest Stickstoff, 10 Vol.-% Xenon/30 Vol.-% Sauerstoff/Rest Stickstoff, 15 Vol.-% Xenon/30 Vol.-% Sauerstoff/Rest Stickstoff, 20 Vol.-% Xenon/30 Vol.-% Sauerstoff/Rest Stickstoff, 25 Vol.-% Xenon/30 Vol.-% Sauerstoff/Rest Stickstoff, 30 Vol.-% Xenon/30 Vol.-% Sauerstoff/Rest Stickstoff, 35 Vol.-% Xenon/30 Vol.-% Sauerstoff/Rest Stickstoff, 40 Vol.-% Xenon/30 Vol.-% Sauerstoff/Rest Stickstoff, 45 Vol.-% Xenon/30 Vol.-% Sauerstoff/Rest Stickstoff, 5 Vol.-% Xenon/21 Vol.-% Sauerstoff/Rest Stickstoff, 10 Vol.-% Xenon/21 Vol.-% Sauerstoff/Rest Stickstoff, 15 Vol.-% Xenon/21 Vol.-% Sauerstoff/Rest Stickstoff, 20 Vol.-% Xenon/21 Vol.-% Sauerstoff/Rest Stickstoff, 25 Vol.-% Xenon/21 Vol.-% Sauerstoff/Rest Stickstoff, 30 Vol.-% Xenon/21 Vol.-% Sauerstoff/Rest Stickstoff, 35 Vol.-% Xenon/21 Vol.-% Sauerstoff/Rest Stickstoff, 40 Vol.-% Xenon/21 Vol.-% Sauerstoff/Rest Stickstoff, 45 Vol.-% Xenon/21 Vol.-% Sauerstoff/Rest Stickstoff,5 Vol.-% Xenon/25 Vol.-% Sauerstoff/Rest Stickstoff, 10 Vol.-% Xenon/25 Vol.-% Sauerstoff/Rest Stickstoff, 15 Vol.-% Xenon/25 Vol.-% Sauerstoff/Rest Stickstoff, 20 Vol.-% Xenon/25 Vol.-% Sauerstoff/Rest Stickstoff, 25 Vol.-% Xenon/25 Vol.-% Sauerstoff/Rest Stickstoff, 30 Vol.-% Xenon/25 Vol.-% Sauerstoff/Rest Stickstoff, 35 Vol.-% Xenon/25 Vol.-% Sauerstoff/Rest Stickstoff, 40 Vol.-% Xenon/25 Vol.-% Sauerstoff/Rest Stickstoff oder 45 Vol.-% Xenon/25 Vol.-% Sauerstoff/Rest Stickstoff. Der Xenon-Gehalt des atembaren Gases kann auch vorteilhaft während der Beatmung variiert werden. Dies geschieht besonders vorteilhaft mit einem gesteuerten Gasdosiergerät, wie es z.B. in der WO 98/31282 beschrieben ist und worauf hiermit Bezug genommen wird. Beispielsweise wird mittels eines Signals eines Blutdruck-Sensors am Patienten das Gasdosiergerät gesteuert. Die Xenon-Menge in dem Atemgas kann auf diese Weise in Abhängigkeit von dem Blutdruck des Patienten dosiert werden. Die atembaren Xenon-haltigen Gasgemische werden besonders vorteilhaft in der Intensivmedizin eingesetzt.

Das eingesetzte Xenon-Gas hat im allgemeinen die natürliche Isotopenzusammensetzung. Die Isotopenzusammensetzung des Xenons kann sich von der natürlichen Isotopenzusammensetzung unterscheiden.
Das Xenon-Gas wird vorzugsweise in hoher Reinheit, wie für medizinische Gase üblich, eingesetzt. Das Xenon-Gas dient als reines Gas oder im Gemisch mit anderen Gasen zur Herstellung eines gasförmigen Arzneimittels.

Gasförmiges Xenon (reines Xenon) wird im allgemeinen als komprimiertes Gas in Druckgasbehältern wie Druckgasflaschen oder Druckdosen bereitgestellt. Auch Xenon-haltige Gasgemische können in Druckgasbehältern bereitgestellt werden. Das gasförmige Arzneimittel kann auch in einem Behälter als verflüssigtes Gas oder Gasgemisch oder in kälteverfestigter Form bereitgestellt werden.

Das Arzneimittel wird in der Regel mit einem Beatmungsgerät mit einer Gasdosiereinheit oder mit einem Anästhesiegerät verabreicht. Das Arzneimittel wird vorteilhaft direkt zur Anwendung aus den reinen Gasen hergestellt, beispielsweise durch Zusammenmischen von Xenon, Sauerstoff und gegebenenfalls einem Inertgas (z. B. mit Hilfe eines Anästhesiegerätes) in unmittelbarer Nähe zum Patienten.

Das Arzneimittel wird in der Regel als feuchtes Gas oder wasserdampfgesättigtes Gas dem Patienten verabreicht.

## Patentansprüche

1. Verwendung von Xenon zur Herstellung eines Herz-Kreislaufmittels, eines Blutdruck erhöhenden Mittels, eines Analgetikums oder eines Sedativums.

2. Verwendung von Xenon zur Herstellung eines Medikamentes zur Behandlung von Herz-Kreislauf-Instabilitäten.

3. Verwendung von Xenon zur Herstellung eines gasförmigen Medikamentes zur Behandlung von Herz-Kreislauf-Instabilitäten von Risikopatienten.

4. Verwendung von Xenon zur Herstellung eines gasförmigen Medikamentes zur Behandlung von Herz-Kreislauf-Instabilitäten bei Schilddrüsenoperationen, Lebertransplantationen, in der Gynäkologie, in der Orthopädie, in der plastischen Chirurgie oder in der Onkologie.

5. Verwendung von Xenon zur Herstellung eines gasförmigen Medikamentes zur Behandlung von Krankheiten des Herzens und/oder des Kreislaufes.

6. Verwendung von Xenon oder Xenon-haltigen Gasgemischen zur Herstellung eines Arzneimittels zur Behandlung von hypotonen Kreislaufzuständen bei Mensch oder Säugetier.

7. Verwendung nach Anspruch 6, **dadurch gekennzeichnet, daß** das Arzneimittel zur Behandlung von hypotonen Kreislaufzuständen dient, die durch Herzinsuffizienz, Hypotonie, Hypovolemie oder Anästhetika bedingter Hypotonie verursacht sind.

8. Verwendung von Xenon zur Herstellung eines Arzneimittels zur Behandlung von Schock oder schockartigen Zuständen.
